# EUROPÄISCHE PATENTANMELDUNG

(11) **EP 0 656 358 A1**
(43) Veröffentlichungstag der Anmeldung: **07.06.1995**
(21) Anmeldenummer: 94118886.4
(22) Anmeldetag: 30.11.1994
(51) Int. Cl.: C07D 471/04

(54) **Verfahren zur Herstellung von Imidazopyridinderivaten**

(30) Priorität: 01.12.1993 CH 3580/93
(71) Anmelder: LONZA AG, CH-3945 Gampel/Wallis (CH)
(72) Erfinder: Kuo, David L. Dr., Brig, Kanton Wallis (CH); Eyer, Martin, Dr., Glis, Kanton Wallis (CH); Roduit, Jean-Paul, Dr., Grône, Kanton Wallis (CH); Wellig, Alain, Ried-Mörel, Kanton Wallis (CH)
(74) Vertreter: Weinhold, Peter, Dr.

(57) **Zusammenfassung**

Es wird ein Verfahren zur Herstellung von Imidazopyridinderivaten der allgemeinen Formel 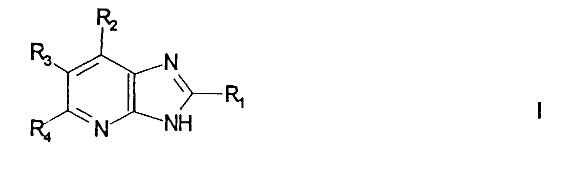
beschrieben.
Dabei wird ein 2-Amino-3-nitropyridin in Gegenwart eines Hydrierkatalysators hydriert und das Hydrierprodukt mit einer gleichzeitig im Reaktionsgemisch vorliegenden Carbonsäure zum Endprodukt kondensiert.
Die Imidazopyridinderivate sind Zwischenprodukte für die Herstellung von Angiotensin-II-Antagonisten.

## Beschreibung

Die Erfindung betrifft ein neues Verfahren zur Herstellung von Imidazopyridinderivaten der allgemeinen Formel 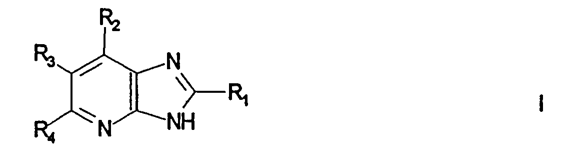
worin
- R₁: Wasserstoff oder eine gegebenenfalls substituierte Alkylgruppe bedeutet und
- R₂, R₃ und R₄: gleich oder verschieden sind und Wasserstoff, eine gegebenenfalls substituierte Alkyl-, Cycloalkyl-, Aryl- oder Aralkylgruppe oder ein Halogenatom bedeuten.

Diese Verbindungen finden Anwendung als Zwischenprodukte für die Herstellung von Angiotensin-II-Antagonisten. (EP-A 0 456 510)

Gemäss EP-A 0 456 510 wird das 5,7-Dimethyl-2-ethylimidazo[4,5b]-pyridin im Gemisch mit 4,6-Dimethyl-2,5-bis(propionamido)pyridin durch Hydrierung eines Isomerengemisches von 2-Amino-3-nitro-4,6-dimethylpyridin und 2-Amino-5-nitro-4,6-dimethylpyridin mit einem Palladiumkatalysator und Wasserstoff, anschliessende Isolation der resultierenden Mischung von 2,3-Diamino- und 2,5-Diaminoisomeren und durch Kondensation dieser Isomerenmischung mit Propionsäure in Gegenwart von Polyphosphorsäure erhalten. Die Reinigung des gewünschten Imidazopyridins erfolgt durch Säulenchromatographie.
Das im Verfahren der EP-A 0 456 510 eingesetzte Isomerengemisch der Aminonitropyridine wird durch Nitrierung des entsprechenden Aminopyridins erhalten.
Beträchtlicher Nachteil dieser bekannten Synthese ist, dass über das gesamte Verfahren ein unerwünschtes Isomer mitumgesetzt wird, welches schliesslich im Endprodukt zu einem schwer abtrennbaren Nebenprodukt führt. Die Umsetzungsbedingungen, insbesondere die lange Hydrierungszeit von 18 Stunden, machen das Verfahren ebenfalls nicht attraktiv für eine Anwendung im technischen Masstab.

Die Aufgabe der Erfindung bestand folglich darin₁ein Verfahren zu entwickeln, das einen einfachen und grosstechnisch umsetzbaren Zugang zu den Imidazopyridinen der allgemeinen Formel I ermöglicht.

Die Aufgabe wird gelöst mit dem Verfahren gemäss Anspruch 1.

Die für die einzelnen Reste R₁ bis R₄ verwendeten Begriffe haben nachfolgende Bedeutung.
Unter der Bezeichnung Alkylgruppe wird eine geradkettige oder verzweigte Alkylgruppe mit zweckmässig 1 bis 6 C-Atomen, vorzugsweise mit 1 bis 4 C-Atomen verstanden. Beispielhaft seien die Methyl-, Ethyl-, n-Propyl, i-Propyl, n-Butyl- oder die t-Butylgruppe genannt.
Unter der Bezeichnung Cycloalkylgruppe wird zweckmässig eine C₃-C₆-Cycloalkylgruppe, wie z. B. eine Cyclopropyl-, eine Cyclobutyl-, eine Cyclopentyl- oder eine Cyclohexylgruppe verstanden.
Die Bezeichnung Aryl umfasst carbocyclische Aromaten, zweckmässig Phenyl oder Naphthyl.
Die Bezeichnung Aralkyl steht für eine arylsubstituierte Alkylgruppe, zweckmässig für eine phenylsubstituierte (C₁ -C₆)-Alkylgruppe, insbesondere für Benzyl.
Unter Halogen wird Fluor, Chlor, Brom oder Jod verstanden, bevorzugtes Halogen ist Chlor.

Die genannten Gruppen, insbesondere die cyclischen Reste, können jeweils einfach oder mehrfach substituiert sein. Geeignete Reste sind z. B. Halogen, Nitro, Amino, Alkylamino, Dialkylamino, Hydroxy, Alkoxy, Alkyl oder Alkanoyl.

Das 2-Amino-3-nitropyridin der allgemeinen Formel 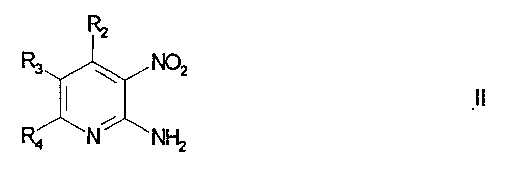
worin R₂, R₃ und R₄ die genannten Bedeutungen hat, wird bevorzugt durch Umsetzung von 1,1-Diamino-2-nitroethen der Formel 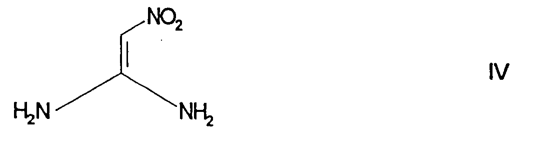
mit einer 1,3-Dicarbonylverbindung der allgemeinen Formel 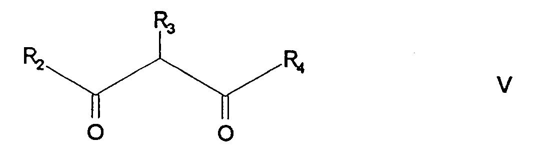
worin R₂, R₃ und R₄ die genannten Bedeutungen hat, erhalten.

Anhand der Reaktion von 1,1-Diamino-2-nitroethen mit Acetylaceton zum 2-Amino-3-nitro-4,6-dimethylpyridin ist diese Umsetzung von Troschütz et al. in Arch Pharm. 325 (1992), 785 - 789 beschrieben worden. Es wird eine Ausbeute von 52% beschrieben.

Durch die Wahl eines geeigneten Lösungsmittels kann die Ausbeute auf über 90% erhöht werden. So erfolgt die Umsetzung bevorzugt bei Rückflusstemperatur in 2-Methoxyethanol. Das entsprechende 2-Amino-3-nitropyridin fällt gemäss diesem Verfahren isomerenrein an und kann ohne zusätzliche Reinigung der erfindungsgemässen Umsetzung zugeführt werden.

Enfindungsgemäss wird das 2-Amino-3-nitropyridin der allgemeinen Formel II in Gegenwart einer Carbonsäure der allgemeinen Formel

R₁COOH III

worin R₁ die genannte Bedeutung hat und einem Hydrierkatalysator mit Wasserstoff hydriert, wobei das gebildete Hydrierprodukt mit der Carbonsäure der allgemeinen Formel III zum Endprodukt kondensiert.
Bevorzugt wird vom 2-Amino-3-nitro-4,6-dimethylpyridin ausgegangen.

Vorteilhaft wird zusätzlich in Gegenwart einer katalytischen Menge einer Säure gearbeitet. Damit erreicht man eine raschere Umsetzung und eine erhöhte Ausbeute.
Geeignete Säure ist z. B. Schwefelsäure, Phosphorsäure oder p-Toluolsulfonsäure. Bevorzugt wird Schwefelsäure in einer Menge von 1 Gew.% bis 20 Gew.%, besonders bevorzugt in einer Menge von ca. 5 Gew.%, bezogen auf das eingesetzte Aminonitropyridin verwendet.

Als Hydrierkatalysator eignet sich Platinoxid oder Palladium auf einem inerten Träger.
Bevorzugt wird 2 Gew.% bis 10 Gew.% Palladium, aufgebracht in einer Menge von 5% bis 10% auf Kohle, verwendet.

Als Carbonsäure der allgemeinen Formel II wird zweckmässig Ameisensäure, Essigsäure, Propionsäure oder Buttersäure, bevorzugt Propionsäure eingesetzt.

In der Regel fungiert die Carbonsäure der allgemeinen Formel III gleichzeitig als. Lösungsmittel. Es ist aber möglich ein inertes Lösungsmittel wie z. B. einen niederen aliphatischen Alkohol oder ein niederes aliphatisches Nitril zuzusetzten.

Die Umsetzung erfolgt zweckmässig bei einem H₂-Druck von 1 bar bis 30 bar und einer Reaktionstemperatur von 100°C bis 150°C. Nach einer Reaktionszeit von in der Regel ca. 2 h bis 10 h kann nach üblicher Aufarbeitung, welche die Abtrennung des Katalysators, die Rückführung der überschüssigen Carbonsäure und eine Extraktion mit einem geeigneten Lösungsmittel umfasst, das gewünschte Imidazopyridinderivat in guter Ausbeute und Qualität erhalten werden.

### Beispiel 1

### Herstellung von 2-Amino-3-nitro-4,6-dimethylpyridin

3 g (29,1 mmol) 1,1-Diamino-2-nitroethen und 11,6 g (17,4 mmol) Acetylaceton wurden in 40 ml 2-Methoxyethanol während 6 h am Rückfluss gehalten. Danach wurde das Lösungsmittel unter Vakuum entfernt, der Rückstand in 20 ml Eiswasser aufgeschlämmt und filtriert. Trocknung des Filtergutes führten zu 4,52 g (93%) des Titelproduktes.
Fp: 165°C
¹H-NMR (CDCl₃, 400 MHz) δ in ppm 2,37 (s, 3H);
2,54 (s, 3H);
6,38 (bs, 2H);
6,44 (s, 1H).

### Beispiel 2

### Herstellung von 5,7-Dimethyl-2-ethylimidazo[4,5b]pyridin

4 g (23,9 mmol) 2-Amino-3-nitro-4,6-dimethylpyridin, 0,2 g (5 Gew.%) 10% Pd/C und 50 ml Propionsäure wurden in einem Autoklaven vorgelegt. Danach wurde 20 bar H₂ aufgepresst und während 7,5 h bei 130°C hydriert. Danach wurde entspannt und 15 ml Propionsäure / Wasser azeotrop destilliert. Dann wurde nochmals 20 bar H₂ aufgepresst und nochmals während 5 h bei 130°C nachreagiert. Nach Abkühlen auf 20°C wurde entspannt und der Katalysator abfiltriert. Das Filtrat wurde eingedampft, der Rückstand mit 25 ml Wasser versetzt und mit Natronlauge auf pH 9 gestellt. Nach Extraktion der wässrigen Phase mit Methylenchlorid konnte aus dem organischen Extrakt das Titelprodukt in einer Rohausbeute von 4,17 g (84,3%) Gehalt GC 84,8% erhalten werden. Kristallisation in Aceton führte zu einem reinen Produkt in Form von hellgelben Kristallen.
Fp: 148°C - 150°C
¹H-NMR (DMSO-d₆, 400 MHz) δ in ppm 1,32 (t, 3H);
2,46 (s, 6H);
2,83 (q, 2H);
6,84 (s, 1H);
12,44 (bs, 1H).

### Beispiel 3

Es wurde entsprechend Beispiel 2 vorgegangen. Zusätzlich wurden 0,2 g (5 Gew.%) Schwefelsäure vorgelegt. Nach der Hydrierung (7,5 h, 20 bar, 130°C) wurde der Katalysator abfiltriert und das Reaktionsgemisch während 3,5 h bei 140°C gerührt. Das resultierende Wasser wurde azeotrop destilliert. Nach Aufarbeitung gemäss Beispiel 2 wurde das Titelprodukt in einer Rohausbeute von 4,21 g (90%) Gehalt GC 89,6% erhalten. Kristallisation aus Aceton führte zu einem reinen Produkt mit einem Gehalt von 97,5%.
Fp: 148°C - 150°C

## Patentansprüche

1. Verfahren zur Herstellung von Imidazopyridinderivaten der allgemeinen Formel 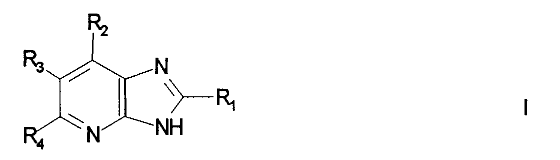 worin R₁ Wasserstoff oder eine gegebenenfalls substituierte Alkylgruppe bedeutet und
R₂, R₃ und R₄ gleich oder verschieden sind und Wasserstoff, eine gegebenenfalls substituierte Alkyl-, Cycloalkyl-, Aryl- oder Aralkylgruppe oder ein Halogenatom bedeuten, dadurch gekennzeichnet, dass ein 2-Amino-3-nitropyridin der allgemeinen Formel 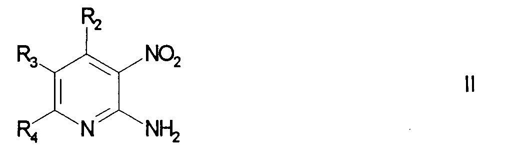 worin R₂, R₃ und R₄ die genannte Bedeutung haben in Gegenwart einer Carbonsäure der allgemeinen Formel
R₁COOH III
worin R₁ die genannte Bedeutung hat und einem Hydrierkatalysator mit Wasserstoff hydriert wird, wobei das gebildete Hydrierprodukt mit der Carbonsäure der allgemeinen Formel III zum Endprodukt kondensiert.

2. Verfahren nach Patentanspruch 1, dadurch gekennzeichnet, dass zusätzlich in Gegenwart einer katalytischen Menge einer Säure gearbeitet wird.

3. Verfahren nach Patentanspruch 2, dadurch gekennzeichnet, dass Schwefelsäure in einer Menge von 1 Gew.% bis 20 Gew.% bezogen auf das 2-Amino-3-nitropyridin der allgemeinen Formel II eingesetzt wird.

4. Verfahren nach einem der Patentansprüche 1 bis 3, dadurch gekennzeichnet, dass als Hydrierkatalysator ein Palladiumkatalysator eingesetzt wird.

5. Verfahren nach einem der Patentansprüche 1 bis 4, dadurch gekennzeichnet, dass die Umsetzung bei einem H₂ Druck von 1 bar bis 30 bar und einer Temperatur zwischen 100°C und 150°C durchgeführt wird.

6. Verfahren nach einem der Patentansprüche 1 bis 5, dadurch gekennzeichnet, dass als Carbonsäure Propionsäure eingesetzt wird.

7. Verfahren nach einem der Patentansprüche 1 bis 6, dadurch gekennzeichnet, dass als 2-Amino-3-nitropyridin der allgemeinen Formel II das 2-Amino-4,6-dimethyl-3-nitropyridin eingesetzt wird.
